# EUROPEAN PATENT SPECIFICATION

(11) **EP 4 426 375 B1**
(45) Date of publication and mention of the grant of the patent: **07.10.2026**
(21) Application number: 23761207.2
(22) Date of filing: 16.08.2023
(51) Int. Cl.: A61M 5/142, A61M 5/168, A61M 5/145, A61M 5/31, A61M 5/315, A61M 5/48

(54) **MEDICAMENT DELIVERY DEVICE**
ARZNEIMITTELABGABEVORRICHTUNG
DISPOSITIF D'ADMINISTRATION DE MÉDICAMENTS

(30) Priority: 19.08.2022 GB 202212140
(43) Date of publication of application: 11.09.2024
(60) Divisional application: 24175238.5 / 4 431 137
(73) Proprietor: Shaily Innovations Ltd, Croydon, Surrey CR0 2LX (GB)
(72) Inventor: HOLMQVIST, Anders, 13237 Saltsjö-Boo (SE)
(74) Representative: Serjeants LLP
(86) International application number: PCT/GB2023/052154
(87) International publication number: WO 2024/038273

(56) References cited:
- WO-A1-2018/067734
- WO-A1-2018/096534
- US-A1- 2019 009 019
- US-A1- 2020 353 160

## Description

### TECHNICAL AREA

The present invention relates to a medicament delivery device and in particular a device that can be attached to a skin surface of a user for administering delivery of medicament.

### BACKGROUND OF INVENTION

There are many medicament delivery devices that have been developed for self-administration of medicament, such as pen-injectors, auto-injectors and the like that are held and operated by the user during the administration of medicament. Other types that have been developed are medicament delivery devices that are attached to the body of a user, so-called wearable devices. Medicament delivery devices may either be developed as multi-use devices that are re-filled or single use, so-called disposable, devices. Regarding wearable devices, there is a challenge to make them as small and discreet as possible and at the same time have a good functionality, especially in relation to automatic functions not performed by the user. The challenge is even more pronounced if the wearable is designed as a disposable device, because of production costs in relation to the complexity in housing important functional and automatic features in a restricted space. Pertinent devices are disclosed in prior art documents US 2019/009019 A1, US 2020/353160 A1 and WO 2018/067734 A1.

### BRIEF DESCRIPTION OF INVENTION

The aim of the present invention is to remedy the drawbacks of the state of the art medicament delivery devices and in particular wearable devices.

This aim is obtained by a medicament delivery device comprising the features according to the independent patent claim.

Aspects of the invention that are preferred but not essential are defined in the dependent patent claims.

According to a main aspect, a medicament delivery device is provided, which comprises an attachment surface for attachment to a skin surface of a user, a drive means, a plunger rod connected to the drive means, a medicament container containing medicament to be pressurised by the plunger rod and the drive means, a valve arrangement fluidly connectable to the medicament container, injection needle fluidly connected to the valve arrangement, and control means operably connected to the valve arrangement for opening and closing a fluid path between the medicament container and the injection needle. The valve arrangement comprises a needle hub, wherein the needle hub comprises the injection needle and a cartridge needle.

The valve arrangement may further comprise a piston movable in a passage by the solenoid, that the injection needle and the cartridge needle are fluidly connected to the passage and that the piston may comprise a fluid path such that, when the piston is in a first position, the fluid connection between the needles is blocked, and when the piston is in a second position, the fluid path fluidly connects the needles. In this regard, the fluid path of the piston may comprise an annular groove.

The drive means and the plunger rod may, upon activation, move the medicament container in contact with the cartridge needle, causing a fluid connection between the medicament container and said cartridge needle.

According to a further aspect, the control means may be designed to operate the valve arrangement for providing a pulsed injection sequence. In this regard the pulsed injection may be controlled regarding pulse frequency and pulse duration.

According to yet an aspect, the control means may comprise a solenoid. In this regard, the control means may further comprise a controller programmed to operate the solenoid.

According to a further aspect, the medicament delivery device may comprise a base member provided with the attachment surface and a unit pivotably connected to the base member. The unit may comprise the drive means, the plunger rod, the medicament container, the valve arrangement, the injection needle and the control means, wherein a pivoting action of the unit in relation to the base member may cause a penetration of the injection needle.

Further, the unit may comprise an attachment surface for attachment to a skin surface of a user when pivoted in relation to the base member.

These and other aspects of, and advantages with, the present invention will become apparent from the following detailed description of the invention and from the accompanying drawings.

### BRIEF DESCRIPTION OF DRAWINGS

In the following detailed description of the invention, reference will be made to the accompanying drawings, of which
- Fig. 1: is a perspective view of a wearable device according to an embodiment of the invention,

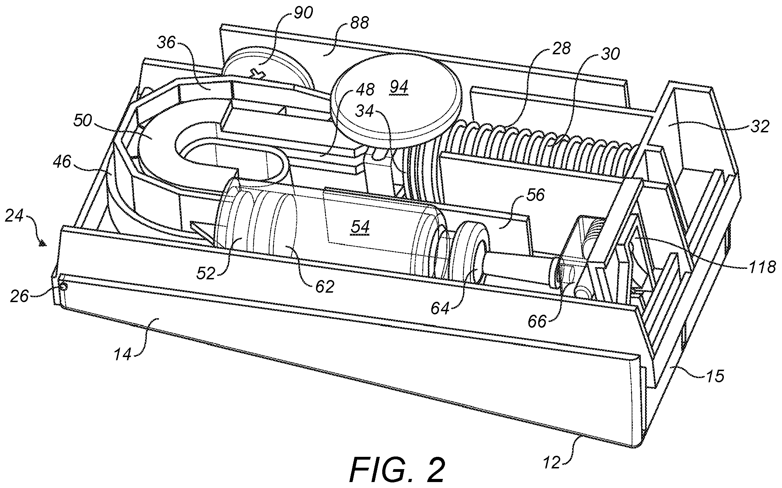

- Fig. 2: is a perspective view of the device of Fig. 1 with a top cover removed,
- Figs. 3-7: are detailed views of components of the device of Fig, 1,
- Figs. 8-10: are cross-sectional views of the device of Fig. 1 in different operational positions,
- Figs. 11-12: are detailed views of a needle hub comprised in the device of Fig. 1,
- Fig. 13: is a detailed view of sensor arrangement comprised in the device of Fig. 1, and
- Figs. 14-16: are perspective views with removed top cover of a locking mechanism for the device of Fig. 1.

### DETAILED DESCRIPTION OF THE INVENTION

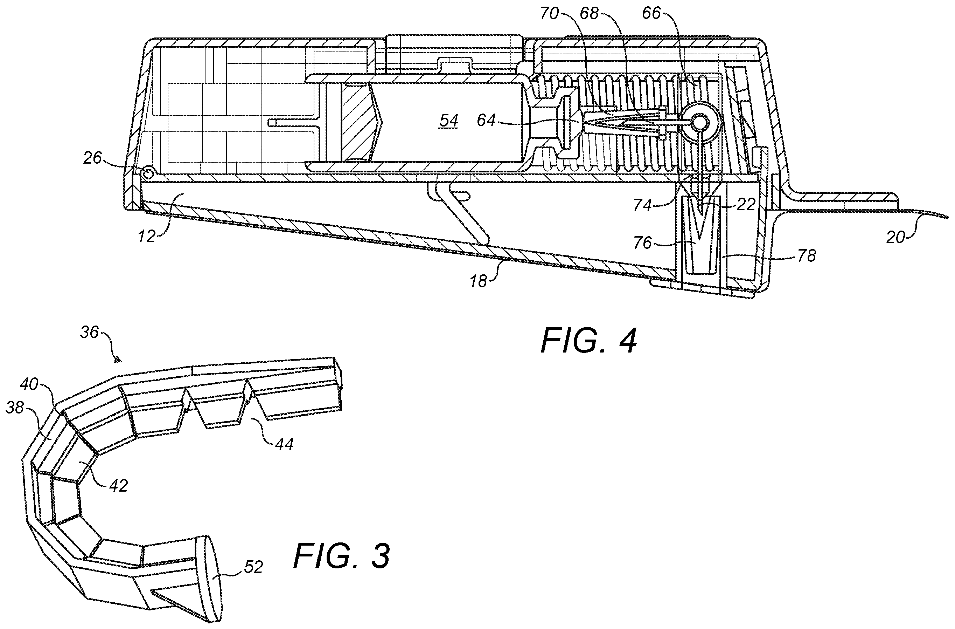

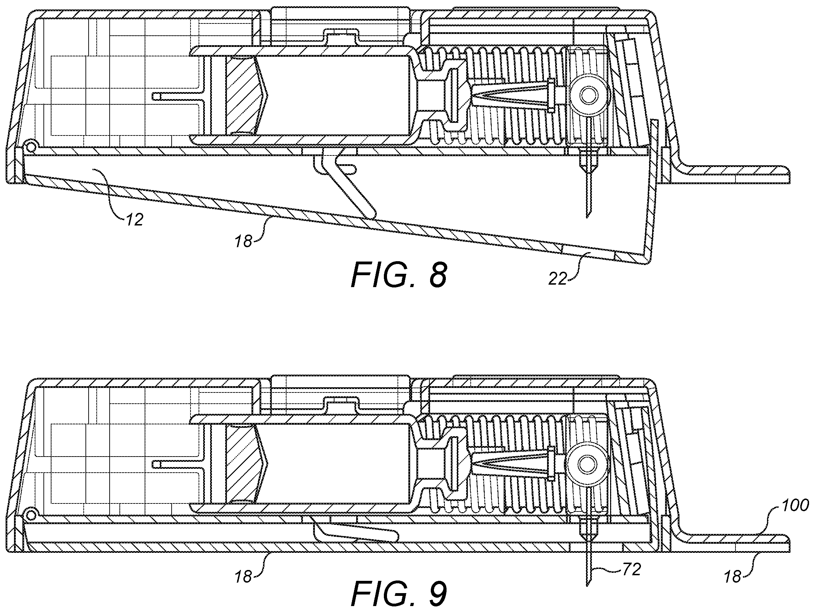

A wearable injector device 10 shown in the drawings comprises a generally rectangular base member 12 having two oppositely arranged side walls 14, an end wall 15 and an underside 16 intended to be in contact with a skin surface of a wearer of the device. The underside 16 is for this purpose preferably provided with an adhesive layer 18, Fig. 4, for attachment to the skin surface. The adhesive layer is before use protected by a protective film 20 that can be peeled off. The underside 16 is further provided with a passage 22, Fig. 8. Further, a generally rectangular base plate 24 is attached to the base member 12 via hinges 26 at one end of the side walls 14 of the base member 12 in order to provide a pivoting action of the base plate 24 in relation to the base member 12 as will be described in detail below.

The base plate 24 is further provided with a power spring 28. The power spring 28 is guided inside by tubular spring guide 30. One end of the power spring 28 is in contact with an end wall 32 of the base plate 24. The opposite end of the power spring 28 is resting on an annular ledge 34 of the spring guide 30. An end surface of the spring guide 30 is to be moved in contact with an end surface of a plunger rod 36, as will be described. The plunger rod 36 is designed with plate-shaped segments 38 oriented generally vertical to the plane of the base plate 24, Fig. 3. The segments 38 are interconnected in series by hinges 40 that could be grooves in the material, forming living hinges. Each segment 38 is further provided with a guide plate 42 oriented generally perpendicular to the surface of the segments 38. The guide plates 42 have bevelled end surfaces forming a V-shaped gap 44 between adjacent guide plates 42.

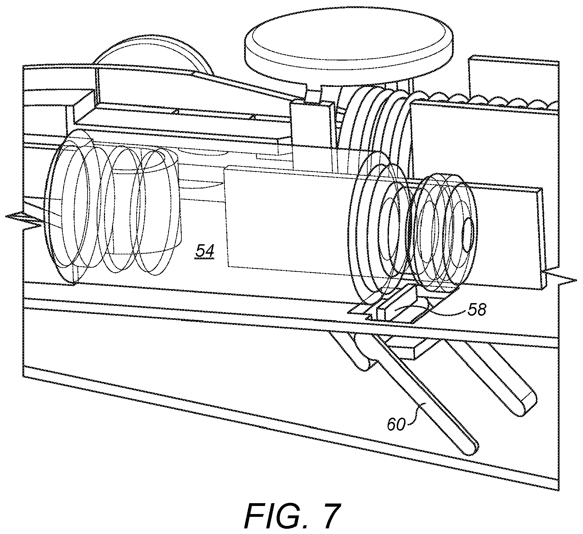

The design of the plunger rod 36 enables it to form a curve, wherein the plunger rod 36 is guided on its outside by a curved support wall 46 in the base plate 24. The guide plates 42 are as seen in Fig. 3 arranged on the inside of the curved plunger rod 36 and fit into a groove 48 on a guide curve 50, which will prevent any transversal movement of the plunger rod 36 during operation, as will be described. The opposite end of the plunger rod 36 from the spring 28 is provided with a generally circular push plate 52. The dimensions of the push plate 52 are such that it will fit into an opening of a generally cylindrical medicament container, such as a cartridge 54, which in turn is placed in a cartridge holder 56 of the base plate 24. Before use, the cartridge 54 is held in an initial position between the push plate 52 of the plunger rod 36 and a stop ledge 58, which is attached to a lever 60, Fig. 7, the function of which will be explained. The cartridge 54 is provided with a movable stopper 62 and has a neck at the opposite end from the opening, which neck is provided with a pierceable septum 64. Thus, a quantity of medicament is contained in the cartridge 54 in the space between the stopper and the septum.

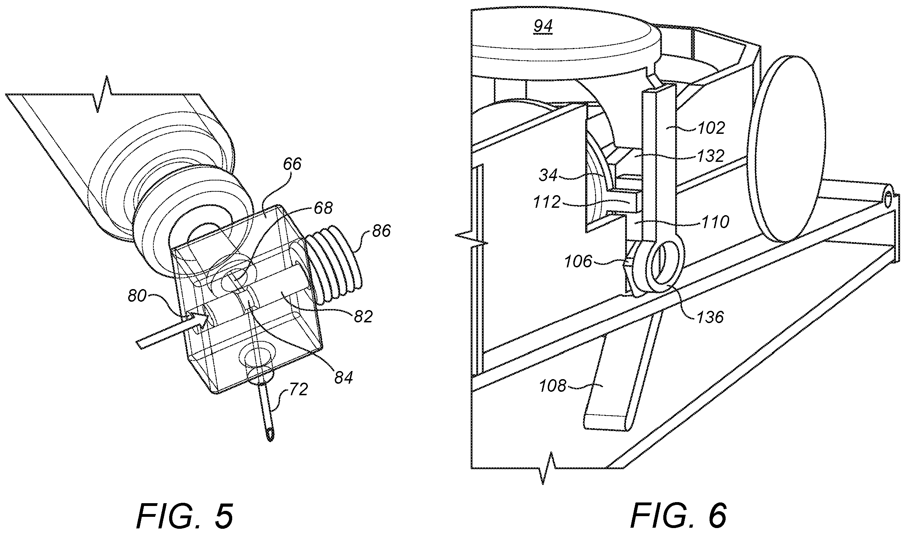

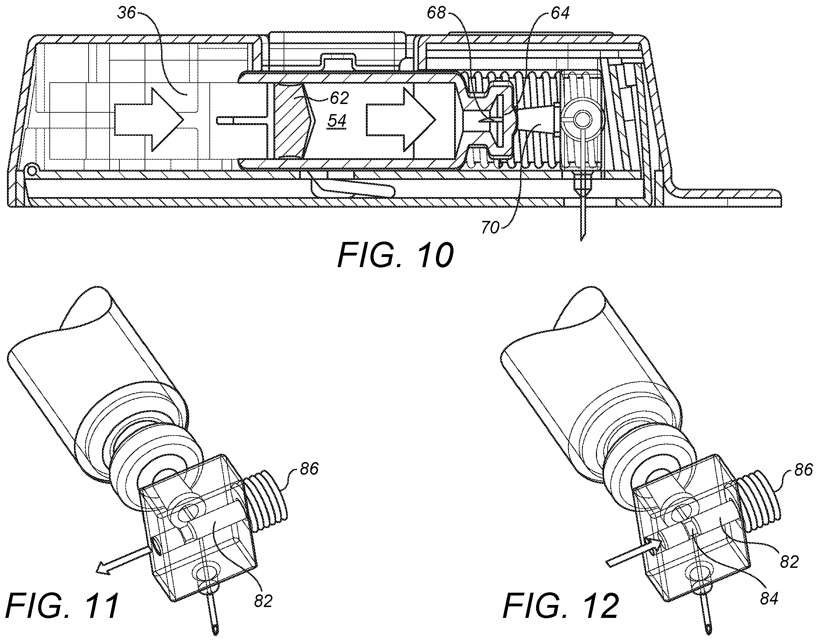

Adjacent the neck end of the cartridge 54, a needle hub 66 is provided, Figs. 4 and 5, attached to or made integral with, the base plate 24, which needle hub is comprised in a valve arrangement. The needle hub 66 is provided with a first hollow needle 68, which is a cartridge needle extending from a side surface thereof, oriented generally horizontal to the plane of the base plate and aligned with the septum 64 of the cartridge 54. The first needle 68 is protected and kept sterile by a flexible sheath 70. The needle hub 66 is further provided with a second hollow needle 72, which is an injection needle extending downwards from a bottom surface of the needle hub 66 and through a passage 74 in the bottom of the base plate 24 and is directed generally perpendicular to the plane of the base plate 24. The second needle 72 is protected and kept sterile by a needle sheath 76, which in turn is connected to a needle cap 78, which needle cap 78 extends from beneath into the passage 22 in the base member 12 and is in an initial position of the device in contact with, or adjacent, the bottom of the base plate 24 as seen in Fig. 4. The needle hub 66 is further provided with a passage 80, wherein openings in the inner ends of the first and the second hollow needles are in fluid communication with the passage 80. In the passage 80 a piston 82 is arranged to be movable. The piston 82 is provided with a fluid communication path, in the embodiment shown as an annular groove 84. The piston 82 is movable between a first position, wherein the openings in the inner ends of the two needles 68, 72 are blocked by the piston 82, and a second position, wherein the groove 84 is aligned with the openings of the needles, providing a fluid path between the two needles, see Figs. 11, 12. The piston 82 is further arranged with suitable sealing members on both sides of the groove 84.

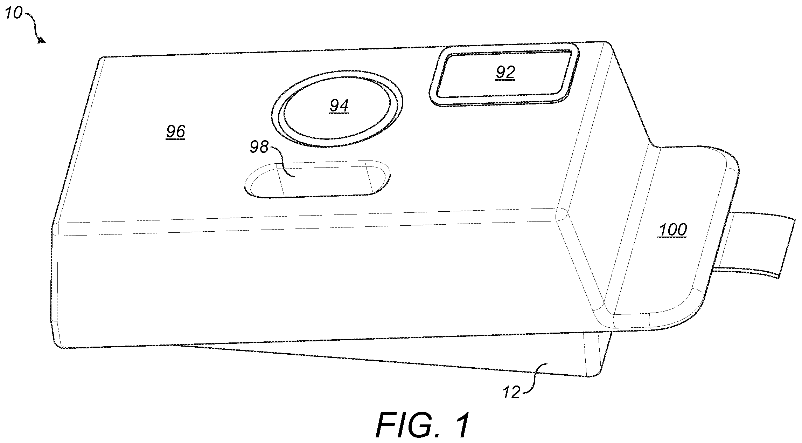

The movement of the piston 82 is provided by a solenoid 86 attached to one end of the piston 82. The solenoid 86 is in turn electrically connected to a controller 88. The controller 88 comprises processor means, memory means and appropriate sensor means. The controller 88 is powered by a battery 90. Further, a display 92 is connected to the controller 88 for showing the status of the device during use as well as a user-operated start button 94. Both the display 92 and the start button 94 may be arranged in a top cover 96 attached to the base plate 24, for providing easy access, Fig. 1. The top cover 96 may further be provided with an opening 98 through which the cartridge and its content is visible. Further, the top cover may be arranged with a lip 100, which in turn may be provided with an adhesive layer on its underside, as will be explained.

The start button 94 is movably arranged in a generally vertical direction by vertical ledges 102 fitted in vertical grooves (not shown) in the base plate 24. Before use, the start button 94 is however blocked from movement by ledges 106 on a lever 108 arranged below the start button 94 in contact with vertical ledges 110 on the start button 94. In this initial position, the start button 94 blocks movement of the spring guide 30, with the tensioned spring 28, by radially extending protrusions 112 on the annular ledge 34 of the spring guide 30 abutting the vertical ledges 110 on the start button, as seen in Fig. 6.

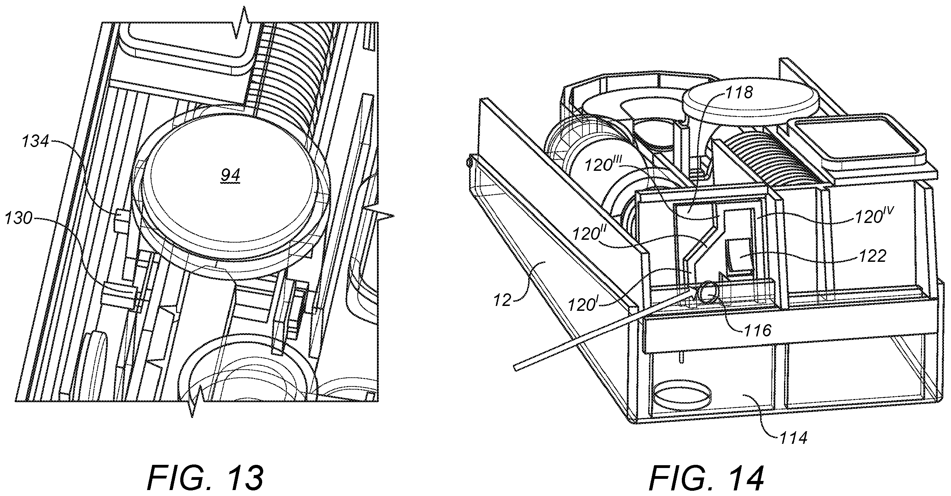

The base member 12 is further provided with an end wall 114 that is positioned inside the end wall 15 of the base plate 24, Fig. 14. An inner surface of the end wall 114 of the base member 12 is arranged with a protrusion 116. Further the base plate 24 is provided with a lock-out plate 118 adjacent the protrusion 116. The surface of the lock-out plate 118 facing the protrusion 116 is provided with guide wall sections 120; a first generally vertically extending section 120^{I}, followed by a second inclined section 120^{II}, in turn followed by a third vertical section 120^{III}. A fourth vertical section 120^{IV} is arranged parallel to the third section. Below the space between the third section and the fourth section, as seen in a vertical direction, a flexible ledge 122 is provided. The function of the lock-out plate will be described below.

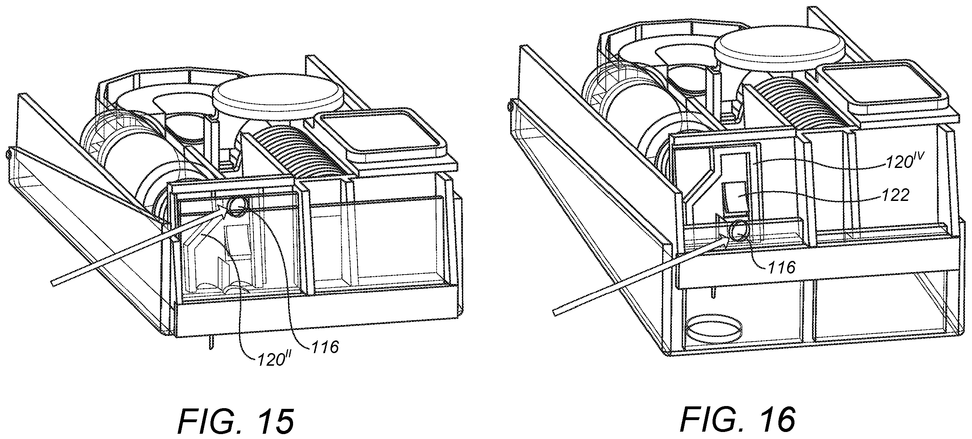

The device is intended to function as follows. When the device 10 is delivered to a user, the unit base plate/ top cover 24/96 is in an inclined position in relation to the base member 12 as seen in Fig. 4. The unit 24/96 is prevented from moving in relation to the base member 12 by the needle cap 78. When the device is to be used, the user removes the protective layer 20 from the underside 16 of the base member and the lip 100 of the top cover 96, whereby the needle cap 78 is also removed, Fig. 8. The user then places and presses the base member 12 of the device 10 against a skin surface for attachment. The pressing of the device and in particular the unit 24/96 will cause a pivoting movement of the unit 24/96 in relation to the base member 12, thereby causing a penetration of the second needle 72 into the skin, Fig. 9. The unit 24/96 is held in this position by the adhesive layer 18 on the lip 100 that attaches to the skin surface. The device is now in position for an injection because the lever 108 blocking the start button 94 has pivoted such that its ledges 106 are out of contact with the start button 94. This pivoting action of the lever 108 is also detected by a sensor 130, Fig. 13, that activates and "wakes up" the electronics of the PCB by providing power from the battery, Fig. 13. Also the lever 60 holding the cartridge 54 has been pivoted so that the cartridge 54 is free to move in the cartridge holder 56. The display 92 is thus activated and may show information that the injection can be started. The pivoting action has further caused the protrusion 116 on the wall of the base member to contact the inclined second section 120^{II} of the lock-out plate 118, thereby pushing the lock-out plate 118 laterally so that the protrusion 116 is in the position shown in Fig. 15. In alternative embodiments, the inner surface of the end wall 114 of the base member 12 could be provided with the lock-out plate 118 and the base plate 24 could be provided with the protrusion 116.

When now the start button 94 is pressed into the top cover, the blocking of the spring guide 30 is removed in that its protrusions 112 can pass cut-outs 132 in the vertical ledges 110 of the start button 94, Fig. 6. The power spring 28 is released, whereby the spring guide 30 can move by its force. The spring guide 30 in turn will act on the segmented plunger rod 36 and the plunger rod 36 will move in contact with the stopper 62. Due to the incompressibility of the medicament, the force from the power spring 28 on the plunger rod 36 will cause the cartridge 54 to move whereby the first needle 68 penetrates the now compressed needle sheath 70 and the septum 64 of the cartridge 54, Fig. 10. The movement of the cartridge 54 is stopped when its front comes in contact with the needle hub 66. The medicament is now pressurised by the plunger rod 36 acting on the stopper 62. Further, the pressing of the start button 94 will be detected by a sensor 134, Fig. 13, which will trigger a start signal of the injection sequence to be initiated. The injection sequence is pre-programmed and stored in the memory means on the controller 88. The electronics will now operate the solenoid 86 to move the piston 82 from the first blocked position, Fig. 11, to the second position, Fig. 12, wherein a flow path between the first needle 68 and the second needle 72 is created by the annular groove 84, and an injection is initiated. Depending on the stored injection sequence, the solenoid 86 will move the piston 82 repeatedly between the first and the second position, thereby for instance creating a pulsed delivery of medicament. The injection sequence may be designed differently depending on various aspects. The pulses may be short or long in duration and/or short or long in frequency. The pressure on the medicament in the cartridge 54 is maintained by the constantly acting spring force. However, the spring force will decline as the spring is extended, and this situation may be handled by altering the pulse opening time in order to keep a constant medicament flow rate over a time span including at least one full pulse cycle. Usually, the solenoid 86 produces a clicking sound when operated, which may be used as an indication to a user that the injection is in progress. Further, the start button 94 may be moved back to its initial position by a spring means 136, Fig. 6, when the user has stopped pressing. A further pressing may be detected by the sensor 134, whereby the injection sequence can be stopped. This may be repeated several times for starting and stopping the injection. Thus, apart from the pre-programmed injection sequence, the user may also control the injection.

When the injection is completed, this may be indicated on the display 92 that it is safe to remove the device from the skin surface. In this regard, there might be a third sensor that is capable of detecting the plunger rod travel position at the end of travel/injection. In addition, the opening 98 may indicate to a user the status of the injection in that the stopper may be visible there. The user then grabs the unit 24/96, whereby the adhesive film of the lip will loosen from the skin. This action will cause the unit 24/96 to pivot back to the initial position in relation to the base member 12, whereby the second needle 72, the injection needle is positioned inside the device. The pivoting movement of the unit 24/96 will further cause the protrusion 116 on the base member 12 to slide along the fourth section 120^{IV} of the lock-out plate 118 and be moved past the flexible ledge 122 and to the position shown in Fig. 16, thereby locking the unit 24/96 in its initial position, preventing any attempts to move the unit back to the injection position and thereby avoiding the risk of unintentional needle sticks. Further pulling of the device will remove it completely from the skin surface. The device is now safe to discard.

It is to be understood that the embodiment described above and shown in the drawings is to be regarded only as a non-limiting example of the invention and that it may be modified in many ways within the scope of the patent claims.

## Claims

1. Medicament delivery device (10) comprising:
- an attachment surface (16, 100) for attachment to a skin surface of a user,
- a drive means (28, 30),
- a plunger rod (36) connected to said drive means (28, 30),
- a medicament container (54) containing medicament to be pressurised by said plunger rod (36) and said drive means (28, 30),
- a valve arrangement fluidly connectable to said medicament container (54),
- an injection needle (72) fluidly connected to said valve arrangement, and
- control means (86, 88) operably connected to said valve arrangement for opening and closing a fluid path between the medicament container (54) and the injection needle (72),
**characterized in that**
said valve arrangement comprises a needle hub (66), the needle hub comprising said injection needle (72) and a cartridge needle (68).

2. Medicament delivery device according to claim 1, wherein:
said valve arrangement comprises a piston (82) movable in a passage (80) by a solenoid (86) of said control means (86, 88),
said injection needle (72) and said cartridge needle (68) are fluidly connected to said passage (80), and
said piston (82) comprises a fluid path (84), wherein, when said piston (82) is in a first position, the fluid connection between the needles is blocked, and when said piston (82) is in a second position, said fluid path (84) fluidly connects said needles.

3. Medicament delivery device according to claim 2, wherein said fluid path (84) of said piston (82) comprises an annular groove (84).

4. Medicament delivery device according to any of the claims 1 to 3, wherein said drive means (28, 30) and said plunger rod (36), upon activation, moves said medicament container (54) into contact with said cartridge needle (68), causing a fluid connection between said medicament container (54) and said cartridge needle (68).

5. Medicament delivery device according to any of the preceding claims, wherein the control means (86, 88) is designed to operate the valve arrangement for providing a pulsed injection sequence.

6. Medicament delivery device according to claim 5, wherein the pulsed injection is controlled regarding pulse frequency and pulse duration.

7. Medicament delivery device according to any of the preceding claims, wherein said control means comprises a solenoid (86).

8. Medicament delivery device according to claim 7, wherein said control means further comprises a controller (88) programmed to operate said solenoid (86).

9. Medicament delivery device according to any of the preceding claims, comprising a base member (12) provided with said attachment surface and a unit (24, 96) pivotably connected to said base member (12), which unit (24, 96) comprises said drive means (28, 30), said plunger rod (36), said medicament container (54), said valve arrangement (66), said injection needle (72) and said control means (86, 88), wherein a pivoting action of said unit (24, 96) in relation to said base member (12) causes a penetration of said injection needle (72).

10. Medicament delivery device according to claim 9, wherein said unit (24, 96) comprises an attachment surface (100) for attachment to a skin surface of a user when pivoted in relation to said base member.

11. Medicament delivery device according to claim 9 or claim 10, further comprising:
a holding lever (60) pivotably connected to the unit (24, 96) and engaging the base member (12), and
a stop ledge (58) attached to the holding lever (60),
wherein before use, the stop ledge (58) holds the medicament container (54) in an initial position, and
wherein said pivoting action of the unit (24, 96) in relation to the base member (12) causes pivoting of the holding lever (60), whereby the stop ledge (58) frees the medicament container (54) to move.

12. Medicament delivery device according to any of claims 9 to 11, further comprising:
a start button (94), which selectively engages the drive means (28, 30),
a blocking lever (108) pivotably connected to the unit (24, 96) and engaging the base member (12), and
at least one blocking ledge (106) on the blocking lever (108),
wherein before use, the start button (94) engages the drive means (28, 30) to prevent the drive means (28, 30) applying pressure to the plunger rod (36), the start button (94) being blocked from movement by contact with the at least one ledge (106), and
wherein said pivoting action of the unit (24, 96) in relation to the base member (12) causes pivoting of the blocking lever (108), whereby the at least one blocking ledge (106) comes out of contact with the start button (94) to free the start button (94) to move out of engagement with the drive means (28, 30).

13. Medicament delivery device according to any of claims 9 to 12,
wherein one of the base member (12) and the unit (24, 96) comprises a protrusion (116) and the other of the base member (12) and the unit (24, 96) comprises a lock-out plate (118),
wherein, during said pivoting action of the unit (24, 96) in relation to the base member (12) to cause a penetration of said injection needle (72), the protrusion (116) causes movement of the lock-out plate (118), whereby, after one use of the device, the lock-out plate (118) prevents the pivoting action of the unit (24, 96) in relation to the base member (12) from being repeated.

14. Medicament delivery device according to claim 13, wherein the lock-out plate (118) prevents the pivoting action of the unit (24, 96) in relation to the base member (12) from being repeated, as a result of the protrusion (116) moving past a flexible ledge (122) on the lock-out plate (118).

## Patentansprüche

1. Medikamentenabgabevorrichtung (10), umfassend:
- eine Befestigungsfläche (16, 100) zur Befestigung an einer Hautoberfläche eines Benutzers,
- ein Antriebsmittel (28, 30),
- eine mit dem Antriebsmittel (28, 30) verbundene Kolbenstange (36),
- einen Medikamentenbehälter (54), der ein Medikament enthält, das durch die Kolbenstange (36) und das Antriebsmittel (28, 30) unter Druck gesetzt werden soll,
- eine Ventilanordnung, die fluidisch mit dem Medikamentenbehälter (54) verbunden werden kann,
- eine Injektionsnadel (72), die fluidisch mit der Ventilanordnung verbunden ist, und
- Steuermittel (86, 88), die mit der Ventilanordnung funktionsfähig verbunden sind, um einen Fluidweg zwischen dem Medikamentenbehälter (54) und der Injektionsnadel (72) zu öffnen und zu schließen,
**dadurch gekennzeichnet, dass**
die Ventilanordnung eine Nadelnabe (66) umfasst, wobei die Nadelnabe die Injektionsnadel (72) und eine Kartuschennadel (68) umfasst.

2. Medikamentenabgabevorrichtung nach Anspruch 1, wobei:
die Ventilanordnung einen Kolben (82) umfasst, der in einem Durchgang (80) durch ein Solenoid (86) der Steuermittel (86, 88) bewegbar ist,
die Injektionsnadel (72) und die Kartuschennadel (68) fluidisch mit dem Durchgang (80) verbunden sind, und
der Kolben (82) einen Fluidweg (84) umfasst, wobei in einer ersten Position des Kolbens (82) die Fluidverbindung zwischen den Nadeln blockiert ist, und in einer zweiten Position des Kolbens (82) der Fluidweg (84) die Nadeln fluidisch verbindet.

3. Medikamentenabgabevorrichtung nach Anspruch 2, wobei der Fluidweg (84) des Kolbens (82) eine ringförmige Nut (84) umfasst.

4. Medikamentenabgabevorrichtung nach einem der Ansprüche 1 bis 3, wobei das Antriebsmittel (28, 30) und die Kolbenstange (36) bei Betätigung den Medikamentenbehälter (54) in Kontakt mit der Kartuschennadel (68) bewegen, wodurch eine Fluidverbindung zwischen dem Medikamentenbehälter (54) und der Kartuschennadel (68) bewirkt wird.

5. Medikamentenabgabevorrichtung nach einem der vorstehenden Ansprüche, wobei die Steuermittel (86, 88) dafür ausgelegt sind, die Ventilanordnung zum Bereitstellen einer gepulsten Injektionssequenz zu betreiben.

6. Medikamentenabgabevorrichtung nach Anspruch 5, wobei die gepulste Injektion hinsichtlich Pulsfrequenz und Pulsdauer gesteuert wird.

7. Medikamentenabgabevorrichtung nach einem der vorstehenden Ansprüche, wobei die Steuermittel ein Solenoid (86) umfassen.

8. Medikamentenabgabevorrichtung nach Anspruch 7, wobei die Steuermittel weiter einen Controller (88) umfassen, der so programmiert ist, dass er das Solenoid (86) betätigt.

9. Medikamentenabgabevorrichtung nach einem der vorstehenden Ansprüche, umfassend ein Basiselement (12), das mit der Befestigungsfläche versehen ist, und eine Einheit (24, 96), die mit dem Basiselement (12) schwenkbar verbunden ist, welche Einheit (24, 96) das Antriebsmittel (28, 30), die Kolbenstange (36), den Medikamentenbehälter (54), die Ventilanordnung (66), die Injektionsnadel (72) und die Steuermittel (86, 88) umfasst, wobei eine Schwenkbewegung der Einheit (24, 96) in Bezug auf das Basiselement (12) ein Eindringen der Injektionsnadel (72) bewirkt.

10. Medikamentenabgabevorrichtung nach Anspruch 9, wobei die Einheit (24, 96) eine Befestigungsfläche (100) zur Befestigung an einer Hautoberfläche eines Benutzers umfasst, wenn sie in Bezug auf das Basiselement geschwenkt wird.

11. Medikamentenabgabevorrichtung nach Anspruch 9 oder Anspruch 10, weiter umfassend:
einen Haltehebel (60), der schwenkbar mit der Einheit (24, 96) verbunden ist und mit dem Basiselement (12) in Eingriff steht, und
eine an dem Haltehebel (60) befestigte Anschlagleiste (58),
wobei, vor der Verwendung, die Anschlagleiste (58) den Medikamentenbehälter (54) in einer Ausgangsposition hält, und
wobei die Schwenkbewegung der Einheit (24, 96) in Bezug auf das Basiselement (12) eine Schwenkbewegung des Haltehebels (60) bewirkt, wodurch die Anschlagleiste (58) den Medikamentenbehälter (54) zur Bewegung freigibt.

12. Medikamentenabgabevorrichtung nach einem der Ansprüche 9 bis 11, weiter umfassend:
einen Startknopf (94), der selektiv mit dem Antriebsmittel (28, 30) in Eingriff steht,
einen Sperrhebel (108), der schwenkbar mit der Einheit (24, 96) verbunden ist und mit dem Basiselement (12) in Eingriff steht, und
mindestens eine Sperrleiste (106) an dem Sperrhebel (108),
wobei vor der Verwendung der Startknopf (94) mit dem Antriebsmittel (28, 30) in Eingriff steht, um zu verhindern, dass das Antriebsmittel (28, 30) Druck auf die Kolbenstange (36) ausübt, wobei der Startknopf (94) durch Kontakt mit der mindestens einen Leiste (106) an der Bewegung gehindert wird, und
wobei die Schwenkbewegung der Einheit (24, 96) in Bezug auf das Basiselement (12) eine Schwenkbewegung des Sperrhebels (108) bewirkt, wodurch die mindestens eine Sperrleiste (106) den Kontakt mit dem Startknopf (94) verliert, um den Startknopf (94) zum Lösen aus dem Eingriff mit den Antriebsmitteln (28, 30) freizugeben.

13. Medikamentenabgabevorrichtung nach einem der Ansprüche 9 bis 12,
wobei eines von dem Basiselement (12) und der Einheit (24, 96) einen Vorsprung (116) umfasst und das andere von dem Basiselement (12) und der Einheit (24, 96) eine Verriegelungsplatte (118) umfasst,
wobei während der Schwenkbewegung der Einheit (24, 96) in Bezug auf das Basiselement (12), die zum Eindringen der Injektionsnadel (72) führt, der Vorsprung (116) eine Bewegung der Verriegelungsplatte (118) bewirkt, wodurch nach einmaliger Verwendung der Vorrichtung die Verriegelungsplatte (118) eine Wiederholung der Schwenkbewegung der Einheit (24, 96) in Bezug auf das Basiselement (12) verhindert.

14. Medikamentenabgabevorrichtung nach Anspruch 13, wobei die Verriegelungsplatte (118) verhindert, dass die Schwenkbewegung der Einheit (24, 96) in Bezug auf das Basiselement (12) wiederholt wird, weil sich der Vorsprung (116) an einer flexiblen Leiste (122) an der Verriegelungsplatte (118) vorbeibewegt.

## Revendications

1. Dispositif (10) d'administration de médicaments comprenant :
- une surface (16, 100) de fixation pour une fixation à une surface de peau d'un utilisateur,
- des moyens (28, 30) d'entraînement,
- une tige (36) de piston reliée auxdits moyens (28, 30) d'entraînement,
- un récipient (54) de médicaments contenant un médicament devant être mis sous pression par ladite tige (36) de piston et lesdits moyens (28, 30) d'entraînement,
- un agencement de valve pouvant être relié de manière fluidique audit récipient (54) de médicaments,
- une aiguille (72) d'injection reliée de manière fluidique audit agencement de valve, et
- des moyens (86, 88) de commande reliés de manière fonctionnelle audit agencement de valve pour ouvrir et fermer un trajet de fluide entre le récipient (54) de médicaments et l'aiguille (72) d'injection,
**caractérisé en ce que**
ledit agencement de valve comprend une embase (66) d'aiguille, l'embase d'aiguille comprenant ladite aiguille (72) d'injection et une aiguille (68) de cartouche.

2. Dispositif d'administration de médicaments selon la revendication 1, dans lequel :
ledit agencement de valve comprend un piston (82) déplaçable dans un passage (80) par un solénoïde (86) desdits moyens (86, 88) de commande,
ladite aiguille (72) d'injection et ladite aiguille (68) de cartouche sont reliées de manière fluidique audit passage (80), et
ledit piston (82) comprend un trajet (84) de fluide, dans lequel, lorsque ledit piston (82) se trouve dans une première position, la connexion fluidique entre les aiguilles est bloquée, et lorsque ledit piston (82) se trouve dans une deuxième position, ledit trajet (84) de fluide relie fluidiquement lesdites aiguilles.

3. Dispositif d'administration de médicaments selon la revendication 2, dans lequel ledit trajet (84) de fluide dudit piston (82) comprend une rainure (84) annulaire.

4. Dispositif d'administration de médicaments selon l'une quelconque des revendications 1 à 3, dans lequel lesdits moyens (28, 30) d'entraînement et ladite tige (36) de piston, lors d'une activation, déplacent ledit récipient (54) de médicaments pour le faire entrer en contact avec ladite aiguille (68) de cartouche, entraînant ainsi une mise en liaison fluidique entre ledit récipient (54) de médicaments et ladite aiguille (68) de cartouche.

5. Dispositif d'administration de médicaments selon l'une quelconque des revendications précédentes, dans lequel lesdits moyens (86, 88) de commande sont conçus pour faire fonctionner l'agencement de valve pour fournir une séquence d'injection pulsée.

6. Dispositif d'administration de médicaments selon la revendication 5, dans lequel l'injection pulsée est commandée concernant la fréquence d'impulsion et la durée d'impulsion.

7. Dispositif d'administration de médicaments selon l'une quelconque des revendications précédentes, dans lequel lesdits moyens de commande comprennent un solénoïde (86).

8. Dispositif d'administration de médicaments selon la revendication 7, dans lequel lesdits moyens de commande comprennent en outre un dispositif (88) de commande programmé pour faire fonctionner ledit solénoïde (86).

9. Dispositif d'administration de médicaments selon l'une quelconque des revendications précédentes, comprenant un élément (12) de base fourni avec ladite surface de fixation et une unité (24, 96) reliée de manière pivotante audit élément (12) de base, laquelle unité (24, 96) comprend lesdits moyens (28, 30) d'entraînement, ladite tige (36) de piston, ledit récipient (54) de médicaments, ledit agencement (66) de valve, ladite aiguille (72) d'injection et lesdits moyens (86, 88) de commande, dans lequel une action de pivotement de ladite unité (24, 96) par rapport audit élément (12) de base entraîne une pénétration de ladite aiguille (72) d'injection.

10. Dispositif d'administration de médicaments selon la revendication 9, dans lequel ladite unité (24, 96) comprend une surface (100) de fixation pour une fixation à une surface de peau d'un utilisateur lorsqu'elle est mise en pivotement par rapport audit élément de base.

11. Dispositif d'administration de médicaments selon la revendication 9 ou la revendication 10, comprenant en outre :
un levier de retenue (60) relié de manière pivotante à l'unité (24, 96) et venant en prise avec l'élément (12) de base, et
un épaulement (58) d'arrêt fixé au levier (60) de retenue,
dans lequel, avant utilisation, l'épaulement (58) d'arrêt maintient le récipient (54) de médicaments dans une position initiale, et
dans lequel ladite action de pivotement de l'unité (24, 96) par rapport à l'élément (12) de base entraîne un pivotement du levier (60) de retenue, de telle sorte que l'épaulement (58) d'arrêt libère le récipient (54) de médicaments pour qu'il se déplace.

12. Dispositif d'administration de médicaments selon l'une quelconque des revendications 9 à 11, comprenant en outre :
un bouton (94) de démarrage, qui vient sélectivement en prise avec les moyens (28, 30) d'entraînement,
un levier (108) de blocage relié de manière pivotante à l'unité (24, 96) et venant en prise avec l'élément (12) de base, et
au moins un épaulement (106) de blocage sur le levier (108) de blocage, dans lequel, avant utilisation, le bouton (94) de démarrage vient en prise avec les moyens (28, 30) d'entraînement pour empêcher les moyens (28, 30) d'entraînement d'appliquer une pression à la tige (36) de piston, le bouton (94) de démarrage étant bloqué pour éviter tout mouvement par contact avec le au moins un épaulement (106) de blocage, et
dans lequel ladite action de pivotement de l'unité (24, 96) par rapport à l'élément (12) de base entraîne un pivotement du levier (108) de blocage, de telle sorte que le au moins un épaulement (106) de blocage sorte du contact avec le bouton (94) d'arrêt pour libérer le bouton (94) d'arrêt afin qu'il se déplace hors de prise avec les moyens (28, 30) d'entraînement.

13. Dispositif d'administration de médicaments selon l'une quelconque des revendications 9 à 12,
dans lequel l'un de l'élément (12) de base et de l'unité (24, 96) comprend une saillie (116) et l'autre de l'élément (12) de base et de l'unité (24, 96) comprend une plaque (118) de verrouillage,
dans lequel, pendant ladite action de pivotement de l'unité (24, 96) par rapport à l'élément (12) de base pour entraîner une pénétration de ladite aiguille (72) d'injection, la saillie (116) entraîne un mouvement de la plaque (118) de verrouillage, de telle sorte qu'après une utilisation du dispositif, la plaque (118) de verrouillage empêche que l'action de pivotement de l'unité (24, 96) par rapport à l'élément (12) de base ne soit répétée.

14. Dispositif d'administration de médicaments selon la revendication 13, dans lequel la plaque (118) de verrouillage empêche que l'action de pivotement de l'unité (24, 96) par rapport à l'élément (12) de base ne soit répétée, à la suite du déplacement de la saillie (116) au-delà d'un épaulement (122) flexible sur la plaque (118) de verrouillage.
